# DEMANDE DE BREVET EUROPEEN

(11) **EP 4 066 847 A1**
(43) Date de publication de la demande: **05.10.2022**
(21) Numéro de dépôt: 21305389.5
(22) Date de dépôt: 29.03.2021
(51) Int. Cl.: A61K 36/82, A61P 29/00, A61K 9/00, A61Q 19/00

(54) **EXTRAIT HYDRO-ALCOOLIQUE DE CAMELLIA JAPONICA ET COMPOSITIONS COSMÉTIQUES LE COMPRENANT**

(71) Demandeur: Chanel Parfums Beauté, 92200 Neuilly-sur-Seine (FR)
(72) Inventeur: COCANDEAU, VINCENT, 93694 PANTIN CEDEX (FR); ROSSIGNOL-CASTERA, Anne, 34400 LUNEL (FR); LEMOINE, Noémie, 34400 LUNEL (FR)
(74) Mandataire: Plasseraud IP

(57) **Abrégé**

L'invention concerne un extrait de Camellia obtenu par extraction d'une poudre de fleurs de *Camellia japonica* au moyen d'un solvant hydro-alcoolique, une composition cosmétique comprenant un tel extrait présentant notamment un effet apaisant pour la peau.

## Description

La présente invention a pour objet un extrait de fleur de *Camellia japonica,* caractérisé en ce qu'il est susceptible d'être obtenu par extraction avec un solvant hydro-alcoolique de fleurs de camellia prétraitées par extraction huileuse, ainsi que l'utilisation dudit extrait en cosmétique, pour apaiser et/ou réduire l'inflammation de la peau humaine.

La peau est la première barrière de protection de l'organisme vis-à-vis de l'environnement. Elle subit donc de nombreuses agressions extérieures qui peuvent conduire à des réactions cutanées d'inconfort voire, en cas de réactions très intenses ou plus graves, à des phénomènes d'irritation et/ou d'inflammation cutanées.

Les réactions cutanées d'inconfort, voir alternativement des réactions d'irritation et/ou d'inflammation de la peau peuvent notamment être induites par le contact avec des produits chimiques tels que les nettoyants, ou provenir d'actions mécaniques tels que le rasage, le gommage, le peeling, l'épilation. Elles peuvent également provenir de l'action de la température, du climat, des rayonnements ultra-violets ou encore de la pollution atmosphérique.

Il existe toujours un besoin pour de nouveaux agents apaisants dans le domaine cosmétique. Il existe également un besoin pour de nouveaux agents, aptes à traiter et/ou diminuer les réactions d'irritation et/ou d'inflammation cutanées dans le domaine dermatologique.

La présente invention a donc pour objet de proposer un nouvel extrait de *Camellia japonica* obtenu au moyen d'un procédé spécifique, et présentant des propriétés cosmétiques apaisantes et anti-inflammatoires.

La Demanderesse a déjà proposé, dans sa demande internationale WO2011/083110, la mise en œuvre d'un extrait hydro-alcoolique de Camellia, pour lutter efficacement contre le dessèchement de la peau. En effet, dans cette demande, l'extrait hydro-alcoolique de Camellia obtenu agit par stimulation de l'expression de l'ARNm de HSP32, stimulation de l'expression de la protéine HSP27 et stimulation de l'expression de la protéine PPAR-β/δ sur les kératinocytes traités.

Toutefois, un tel extrait ne permet pas d'agir sur les gènes impliqués dans les voies liées à l'inflammation dans les kératinocytes humains normaux, de sorte qu'il ne permet pas de réduire l'inflammation cutanée ni d'agir comme agent apaisant.

Il est ainsi du mérite de la demanderesse d'avoir mis au point un procédé particulier de préparation d'un nouvel extrait hydro-alcoolique de fleur de *Camellia japonica* présentant de propriétés apaisantes pour la peau inconnues des extraits de fleur de *Camellia japonica* actuellement sur le marché, et une action anti-inflammatoire pour la peau par inhibition de l'expression des gènes impliqués dans les voies liées à l'inflammation dans les kératinocytes humains normaux.

La présente invention a ainsi pour objet, selon un premier aspect, un procédé particulier de préparation d'un extrait hydro-alcoolique de fleurs de *Camellia japonica,* comprenant les étapes suivantes :
i. l'imprégnation d'une poudre de fleurs de Camellia par un corps gras ou un mélange de corps gras à une température supérieure à la température de fusion dudit corps gras et sous atmosphère dépourvue ou essentiellement dépourvue d'oxygène, de préférence une huile, puis séparation pour obtenir d'une part un extrait huileux de fleurs de Camellia et d'autre part une poudre résiduelle des fleurs ainsi extraites,
ii. l'imprégnation de la poudre résiduelle des fleurs obtenue à l'étape i. au moyen d'un solvant d'extraction à base d'un mélange d'alcool et d'eau sous atmosphère dépourvue ou essentiellement dépourvue d'oxygène,
iii. l'extraction de la dite poudre résiduelle de fleurs par ultrasons, notamment basse fréquence, et sous atmosphère dépourvue ou essentiellement dépourvue d'oxygène,
iv. la macération des drèches dans le solvant d'extraction et sous atmosphère dépourvue ou essentiellement dépourvue d'oxygène,
v. la clarification, par exemple par microfiltration, de l'extrait hydro-alcoolique de fleurs de *Camellia.*

L'invention a également pour objet, selon un second aspect, un extrait hydro-alcoolique de fleurs de *Camellia japonica,* comprenant majoritairement un mélange de sucres, de camellioside A et de caméllioside B, de préférence obtenu au moyen du procédé précédemment décrit.

L'invention a encore pour objet, selon un troisième aspect, une composition cosmétique comprenant, dans un milieu physiologiquement acceptable, au moins un tel extrait de fleurs de *Camellia japonica.*

Enfin, l'invention a pour objet, selon un quatrième aspect, l'utilisation cosmétique non thérapeutique dudit extrait de fleur de *Camellia japonica,* comme actif apaisant et/ou anti-inflammatoire.

### Camellia japonica

Le Camellia est un genre de plantes à fleurs de la famille des Théacées, originaire d'Asie orientale et méridionale de l'est depuis la chaîne de l'Himalaya jusqu'au Japon et en Indonésie. Les fleurs de Camellia sont reconnues entre autres pour leurs propriétés antibactériennes, anti-oxydantes, anti-inflammatoires, astringentes et toniques. Le nombre d'espèces que contient le genre diffère suivant les botanistes, et varie entre 100 à 250 espèces. On peut notamment citer les fleurs de couleur blanche, notamment la variété *Alba plena,* des fleurs de *Camellia japonica* de couleur rouge très foncé du type rouge chocolat à rouge noir, notamment les variétés *Black magic, Kuro tsubaki, Black domino, Koronkoku, Kon wabisuke, Kuro wabisuke, Murasaki-no-ue, Sir Victor Davis,* des fleurs de camellias hybrides de *Kuro tsubaki* dont les noms de variété sont *Night rider* ou *Black opal.* Les variétés *Black magic* et *Night rider* sont les variétés de *Camellia japonica* les plus courantes en France. Les couleurs des fleurs de camélia peuvent varier en fonction du pH, des métaux et des métalloïdes présents dans le sol ou substrat. Les camellias fleurissent généralement de mi-février à avril. Il est toutefois possible d'obtenir des floraisons dès octobre par traitement hormonal.

L'extrait selon l'invention est obtenu à partir des fleurs de *Camellia japonica,* et de préférence, à partir des fleurs de couleur blanche, notamment de la variété *Alba plena.* De préférence, les fleurs de *Camellia japonica* utilisées dans l'invention sont cultivées en France.

Les fleurs de *Camellia japonica* se présentent de préférence sous forme de poudre dispersible. Par dispersible, on entend que la poudre de fleurs de Camellia se présente sous une forme dissociée apte à être dispersée finement, et par exemple, la matière première est sous forme particulaire et de préférence pulvérulente. Les fleurs de Camellia fraiches sont, par exemple, dans un premier temps, isolées des tiges puis ouvertes et mises à plat sur des claies. Elles sont ensuite déshydratées en conditions douces, soit à température ambiante à l'abri de la lumière soit dans un séchoir ventilé à une température inférieure à 35°C. Les fleurs sont de préférence séchées jusqu'à obtention d'une teneur en matière sèche supérieure à 80% et préférentiellement supérieure à 85%.

Les fleurs sont ensuite réduites en poudre dispersible par tout procédé de broyage classiquement connu de l'homme du métier, par exemple à température ambiante dans un broyeur à couteaux ou, selon un mode de réalisation préféré, par broyage à basse température. Pour un broyage à basse température, les fleurs sont de préférence refroidies à -80°C dans une enceinte fermée thermostatée et immédiatement broyées dans un mixer à hélices à une température comprise entre -20 et -80°C, pour obtenir une poudre fine et régulière. La cryogénisation permet avantageusement d'assurer un broyage efficace, d'obtenir une poudre homogène, de limiter la coloration de la poudre et de garantir une meilleure conservation des propriétés apaisantes des molécules contenues dans les fleurs.

De préférence, la poudre dispersible de fleurs de *Camellia japonica* mise en œuvre pour la préparation de l'extrait selon l'invention présente une taille moyenne de particules inférieure à 500 µm, préférentiellement inférieure à 300 µm. La poudre de fleurs de *Camellia japonica* présente une odeur florale douce et une couleur allant de blanc crème à rouge brun.

### Procédé de préparation d'un extrait hydro-alcoolique de fleurs de Camellia japonica

Selon une caractéristique essentielle de l'invention, les étapes i., ii., iii., et iv. du procédé de préparation de l'extrait de Camellia sont conduites en atmosphère dépourvue ou essentiellement dépourvue d'oxygène. Ceci signifie que l'on travaille sous gaz ou atmosphère inerte ou sous vide ou vide partiel. La teneur résiduelle en oxygène doit être suffisamment basse pour ne pas provoquer de réactions d'oxydation sensibles à la température du traitement thermique. On peut donc conduire ces étapes sous atmosphère inerte, par exemple sous azote et de préférence sous balayage continu d'azote, permettant l'extraction de l'oxygène présent ou susceptible de se former. On peut employer un réacteur clos avec une extraction continue de l'oxygène par flux d'azote. On peut aussi faire un barbotage d'azote, associé au flux d'azote, au moins au début du traitement thermique. On peut aussi conduire ces étapes sous vide. Procéder ainsi confère un avantage supplémentaire, à savoir l'entraînement des matières volatiles avec un effet de désodorisation partielle du mélange.

Selon un mode préféré de réalisation, les étapes i., ii., iii., et/ou iv. sont conduites en réacteurs fermés en l'absence de lumière ou de tout rayonnement oxydant tel que les UV.

### Etape i. d'imprégnation des fleurs de Camellia par un corps gras

L'invention a pour objet un procédé de préparation d'un extrait hydro-alcoolique de fleurs de Camellia, comprenant une première étape i. d'imprégnation d'une poudre de fleurs de Camellia par un corps gras ou un mélange de corps gras à une température supérieure à la température de fusion dudit corps gras et sous atmosphère dépourvue ou essentiellement dépourvue d'oxygène, de préférence une huile, puis séparation pour obtenir d'une part un extrait huileux de fleurs de Camellia et d'autre part les drèches des fleurs ainsi extraites.

Un procédé permettant la préparation d'un extrait huileux de Camellia conduisant, à l'obtention d'une poudre résiduelle de fleurs de Camellia (co-produit) est par exemple décrit en détails dans la demande WO 2016/016515.

En particulier, le corps gras utilisé comme solvant d'extraction est préférentiellement d'origine végétale, et peut être une huile végétale liquide à température ambiante (20-25°C), un beurre végétal présentant un point de fusion compris entre 25 et 40°C, ou une cire végétale présentant un point de fusion supérieur à 40°C. Selon un mode préféré de réalisation, le corps gras utilisé comme solvant d'extraction est une huile végétale liquide à une température inférieure à la température ambiante, et en particulier liquide à une température d'environ 20°C.

A titre d'exemple d'huiles pouvant être utilisées pour l'obtention de l'extrait selon l'invention, on peut citer l'huile de camélia, l'huile de colza, l'huile de tournesol, l'huile d'olive, l'huile de sésame, l'huile de noyaux d'abricot, l'huile de pépin de raisin, l'huile d'amande douce, l'huile de carthame, l'huile de noisette, l'huile d'argan, l'huile de rosier muscat, l'huile d'onagre, l'huile de bourrache, la cire liquide de jojoba, et leurs mélanges. Il sera choisi préférentiellement une huile source d'acides gras polyinsaturés oméga 6 pouvant jouer un rôle positif sur la fluidité membranaire et sur l'hydratation de la peau.

L'étape i. est conduite à une température supérieure ou égale au point de fusion du corps gras ou du mélange de corps gras utilisé. En particulier, la température est avantageusement comprise entre cette température de fusion et la température de fusion +20°C, de préférence +10°C. La température ambiante (20-25°C) est parfaitement adaptée pour les corps gras tels que les huiles liquides à cette température. La durée de l'étape i. peut être comprise entre 1 et 48 heures, de préférence entre 5 et 40 heures, plus préférentiellement entre 12 et 36 heures, encore plus préférentiellement entre 20 et 30 heures, et selon un mode particulièrement préféré de réalisation, la durée de l'étape i. d'imprégnation est d'environ 24 heures.

L'étape i. est réalisée sous atmosphère dépourvue ou essentiellement dépourvue d'oxygène, et de préférence sous atmosphère saturée d'azote.

Le procédé permettant l'imprégnation de la poudre de fleurs de Camellia par un corps gras peut également comprendre une étape i.2. de micro-dispersion de la poudre de fleurs de Camellia dans le corps gras à une température supérieure à la température de fusion dudit corps, sous atmosphère dépourvue ou essentiellement dépourvue d'oxygène.

Cette étape i.2. peut être opérée par traitement du mélange par cavitation ultrasonore. La cavitation et la dispersion sous ondes ultrasonores sont de préférence réalisées dans un réacteur fermé équipé d'un générateur d'ultrasons à basse fréquence de cavitation, notamment inférieure à 100 kHz et de préférence de l'ordre de 20 à 30 kHz.

La durée du traitement sous ultrasons à l'étape i.2. est notamment comprise entre 2 et 30 minutes, de préférence entre 10 et 20 minutes.

L'étape i.2. est avantageusement conduite à température ambiante ou à une température supérieure au point de fusion du corps gras ou des corps gras utilisés. La température est avantageusement comprise entre cette température de fusion et la température de fusion +20°C, de préférence +10°C. La température ambiante (20-25°C) est parfaitement adaptée pour les huiles liquides à cette température.

Le procédé d'obtention de l'extrait de fleur de Camellia selon l'invention peut également comprendre une étape i.3. de chauffage du mélange obtenu à l'étape i. ou i.2. de ladite poudre de fleurs de Camellia avec le ou lesdits corps gras à une température comprise entre 80 et 180°C pendant une durée comprise entre 1 et 10 minutes, sous atmosphère dépourvue ou essentiellement dépourvue d'oxygène.

Dans un mode de réalisation préféré, la température de l'étape i.3. est comprise entre 80 et 150°C, de préférence entre 90 et 130°C.

L'étape i.3. de chauffage est mise en œuvre pendant une très courte durée allant de 1 à 10 minutes, de préférence de 1 à 5 minutes, et plus préférentiellement de 1 à 3 minutes, cette durée correspondant au temps de maintien de la température de traitement une fois cette température atteinte. Le temps de montée en température est également très court, notamment inférieur ou égal à 5 minutes, de préférence de 1 à 5 min, et plus préférentiellement de 1 à 3 minutes.

Tout système de chauffage thermique rapide peut être utilisé, et dans un mode préféré de réalisation, le traitement thermique est assuré par des micro-ondes. L'utilisation d'une source micro-ondes dans un réacteur fermé permet d'atteindre en un temps très court les températures souhaitées. Le chauffage à haute température permet par ailleurs d'accroître le pouvoir de solubilisation du corps gras utilisé et favorise le contact entre la poudre de fleur de Camellia et ledit corps gras, favorisant ainsi le rendement de l'extraction. Selon un mode préféré de réalisation, le générateur de micro-ondes mis en œuvre pour le chauffage de l'étape i.3. présente une puissance utile allant de 500 à 10000 Watts par kilogramme de mélange, de préférence de l'ordre de 700 à 1500 Watts par kilogramme de mélange, et plus préférentiellement de l'ordre de 1000 Watts par kilogramme de mélange.

Selon un mode particulier de réalisation, on ajoute lors de l'étape i.3. ou juste avant, un composé piégeur ou réducteur d'oxygène. Il est possible ainsi d'ajouter de la vitamine C, sous forme d'acide ascorbique pur, de sel tel que l'ascorbate de sodium ou de palmitate d'ascorbyle, de l'acide citrique ou de l'acide lactique sous forme libre ou ester ou des lécithines, ou encore une combinaison de ces composés. Il sera ajouté une quantité individuelle de 0,01 à 1 % en poids dans le mélange, préférentiellement de 0,1 à 0,5 % en poids dans le mélange.

Les étapes i., i.2. et/ou i.3. sont avantageusement conduites en l'absence de lumière ou de tout rayonnement oxydant tels que les UV pour limiter les risques de photo-oxydation et de dégradation des molécules photosensibles.

Les étapes i., i.2. et/ou i.3. peuvent être réalisées dans un réacteur fermé avec ou sans agitation du mélange et préférentiellement avec agitation.

Selon un mode de réalisation le procédé consiste en une séquence combinée des étapes i., i.2. et/ou i.3. l'ordre des étapes i.2. et i.3 étant indifférent, chaque étape étant réalisée au minimum une fois chacune.

A titre de finition de l'étape i., une ou plusieurs étape(s) i.4. de séparation de l'extrait huileux et de la poudre résiduelle de fleurs de *Camellia japonica* sont mises en œuvre. Ces méthodes de séparation peuvent par exemple être choisies parmi la filtration, la décantation, la centrifugation, l'essorage, ou une association de ces techniques. De préférence, la séparation est effectuée par centrifugation ou filtration. Cette étape de séparation peut être réalisée à une température comprise entre 40 et 60°C dans une essoreuse munie d'une toile filtrante de porosité inférieure à 10 µm et préférentiellement inférieure ou égale à 5 µm et à une vitesse comprise entre 500 et 2500 tours par min et de préférence entre 1000 et 1500 tours / min.

La ou les étapes de séparation permettent de séparer l'extrait huileux de la poudre résiduelle de fleurs de *Camellia japonica* et simultanément d'obtenir un extrait huileux limpide à l'œil et exempt de microparticules en suspension.

A l'issue des étapes i., i.2., i.3. et/ou i.4., on obtient d'une part un extrait huileux de fleurs de Camellia et d'autre part une poudre résiduelle de fleurs de Camellia, ayant donc préalablement subi uneextraction huileuse.

Cette poudre résiduelle de fleurs utilisée dans le procédé selon la présente invention, se présente comme un tourteau végétal gras, ou un gâteau de filtration, d'aspect homogène et coloré en brun, facilement dispersible dans un liquide.

### Etape ii. d'imprégnation de la poudre résiduelle de fleurs de Camellia

A l'issue de l'étape i., le procédé selon l'invention met en œuvre une étape ii. d'imprégnation de la poudre résiduelle de fleurs obtenue à l'étape i. (tourteau) au moyen d'un solvant d'extraction à base d'un mélange d'alcool et d'eau sous atmosphère dépourvue ou essentiellement dépourvue d'oxygène.

L'imprégnation de la poudre résiduelle de fleurs de Camellia peut être effectuée par extraction alcoolique à l'aide d'au moins un monoalcool tel que l'éthanol, le méthanol ou l'isopropanol et/ou d'au moins un glycol tel que le propylène glycol (propanediol) ou le dipropylène glycol, mélangé(s) à de l'eau.

L'extraction est réalisée en l'absence de tout autre solvant que l'eau et les alcools.

Le solvant mis en œuvre à l'étape ii. est de préférence un mélange de polyol et d'eau, et plus préférentiellement de propanediol et d'eau.

Selon un mode préféré de réalisation, le ratio volumique alcool:eau du solvant d'extraction mis en œuvre à l'étape ii. est compris entre 95%/5% et 50% / 50 %, de préférence entre 90%/10% et 70%/30%, et plus préférentiellement entre 85%/15% et 75%/25%.

L'imprégnation est généralement réalisée en dispersant ou en agitant doucement la poudre résiduelle de fleurs de Camellia dans un ou plusieurs des solvants cités ci-dessus à température ambiante, soit entre 20 et 25°C, pendant une durée d'environ 15 min à 2 h et de préférence de 30 min à 1 heure.

Le rapport (en volume/poids) solvant/matière peut par exemple être compris entre 1:1 et 20:1 et de préférence entre 5:1 et 10:1.

Cette étape de dispersion et de mélange est usuelle dans le domaine des extraits de plantes, et l'homme du métier est à même d'en ajuster les paramètres et de choisir les outils d'agitation et dispersion, sur la base de ses connaissances générales.

### Etape iii. l'extraction assistée par ultrasons de la poudre résiduelle de fleurs

Le procédé selon l'invention met ensuite en œuvre une étape d'extraction de la poudre résiduelle de fleurs par cavitation ultrasonore et sous atmosphère dépourvue ou essentiellement dépourvue d'oxygène.

Cette étape iii. permet non seulement une micro-dispersion homogène de la poudre résiduellede fleurs de Camellia dans le solvant d'extraction, mais également la rupture des cellules végétales des dites fleurs ce qui libère les molécules contenues dans ces cellules... La cavitation et la dispersion sous ondes ultrasonores sont de préférence réalisées dans un réacteur fermé équipé d'un générateur d'ultrasons à basse fréquence de cavitation, notamment inférieure à 100 kHz et de préférence de l'ordre de 20 à 30 kHz.

La durée du traitement sous ultrasons est notamment comprise entre 15 et 60 minutes, de préférence entre 30 et 40 minutes.

L'étape iii. est avantageusement conduite à température ambiante (entre 20 et 25°C)et sous atmosphère dépourvue ou essentiellement dépourvue d'oxygène.

### Etape iv. La macération de la poudre résiduelle de fleurs de Camellia

Une fois l'extraction assistée par ultrasons effectuée, le procédé selon l'invention met en œuvre une étape iv. de macération de la poudre résiduelle de fleurs dans le solvant d'extraction, qui permet une diffusion des molécules extraites dans le solvant hydro-alcoolique.

Cette étape consiste à laisser reposer le mélange de la poudre de fleurs et du solvant d'extraction pendant une durée comprise entre 30 min et 3 heures et de préférence entre 1 heure et 2 heures.

L'étape iv. est avantageusement conduite à température ambiante (entre 20 et 25°C) et sous atmosphère dépourvue ou essentiellement dépourvue d'oxygène.

### Etape v. la clarification de l'extrait hydro-alcoolique

A titre de finition, le procédé selon l'invention comprend une ou plusieurs étape(s) de clarification de l'extrait hydro-alcoolique.

On entend par clarification toutes les séparations mécaniques connues de l'homme du métier.

Elles peuvent par exemple être choisies parmi la filtration, la décantation, la centrifugation, l'essorage, ou une association de ces techniques.

De préférence, la séparation est effectuée par filtration, plus préférentiellement encore par microfiltration en deux temps :
- une première microfiltration centrifuge dite essorage sur une toile filtrante de porosité inférieure à 5 microns et de préférence inférieure ou égale à 1 micron, puis
- une seconde microfiltration clarifiante sur une plaque de filtration de porosité inférieure à 1 micron et de préférence inférieure ou égale à 0,2 micron.

On entend par microfiltration centrifuge l'utilisation simultanée de la centrifugation et de la filtration. Le mélange est placé dans un bol inox muni d'une toile filtrante à travers laquelle passe la phase liquide qui est alors clarifiée alors que la phase solide reste à l'intérieur du panier. Cette opération peut être réalisée à une température comprise entre 20 et 25°C dans une essoreuse munie d'une toile filtrante de porosité inférieure à 5 µm et préférentiellement inférieure ou égale à 1 µm et à une vitesse comprise entre 500 et 2500 tours / min et de préférence entre 1000 et 1500 tours / min.

On entend par microfiltration clarifiante l'utilisation d'une plaque en matériau inerte ayant une porosité inférieure ou égale à 1 micron pour assurer la séparation de très fines particules présentes en suspension dans un liquide. Si la porosité de la plaque est de 0,2 microns ou inférieure, alors la filtration permet d'éliminer aussi les microorganismes présents dans le liquide et la filtration est dite stérilisante. Cette opération peut être réalisée à une température comprise entre 20 et 25°C sur un filtre à plaques muni d'une plaque de filtration de porosité de 0,2pm. Le liquide peut être poussé par une pompe ou une surpression d'azote.

Les étapes de clarification permettent l'obtention d'un extrait hydro-alcoolique à la fois substantiellement limpide à l'œil et exempt de microparticules en suspension.

### Extrait hydro-alcoolique de fleurs de Camellia japonica

L'invention objet de la présente demande couvre également un extrait hydro-alcoolique de fleurs de Camellia, comprenant majoritairement un mélange de sucres, de camellioside A et de caméllioside B :

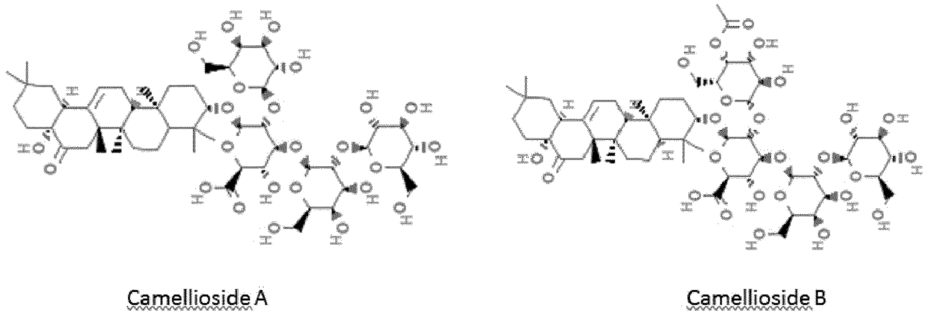

Les sucres présents dans l'extrait sont de préférence choisis parmi le glucose, le fructose et un mélange de ceux-ci, et de préférence, un mélange de glucose et de fructose.

L'extrait hydro-alcoolique de fleurs de *Camellia japonica* objet de la présente invention comprend des sucres, et des camelliosides en une proportion massique d'environ 2 :1 dans l'extrait.

L'extrait hydro-alcoolique de fleurs de *Camellia japonica* objet de la présente invention peut notamment être obtenu au moyen du procédé précédemment décrit.

L'extrait de fleur de *Camellia japonica* est utilisé selon l'invention à des fins cosmétiques non thérapeutiques, pour un effet apaisant et/ou anti-inflammatoire pour la peau.

L'effet apaisant et/ou anti-inflammatoire de l'extrait selon l'invention peut notamment être observé par une diminution de l'expression des gènes impliqués dans les voies liées à l'inflammation dans les kératinocytes humains normaux, selon des techniques habituelles bien connues de l'homme du métier.

Les gènes impliqués dans les voies liées à l'inflammation dans les kératinocytes humains normaux sont notamment B2M (beta-2-microglobulin), HLA-B (major histocompatibility complex, class I, B), IL1A (interleukin 1 alpha), SERPINE1 (serpin family E member 1), ACVR1B (activin A receptor type 1B), CEBPB (CCAAT enhancer binding protein beta), TNF (tumor necrosis factor), HLA-DQB1 (major histocompatibility complex, class II, DQ beta 1).

### Composition cosmétique

La présente invention a encore pour objet une composition cosmétique comprenant, dans un milieu physiologiquement acceptable, au moins un extrait de fleur de *Camellia japonica.*

De façon préférée, la composition selon l'invention, contenant l'extrait de fleur de *Camellia japonica,* est appliquée sur une peau irritée, de préférence non pathologique. Elle peut être avantageusement appliquée sur la peau du visage, du cou, des mains et éventuellement du décolleté ou en variante sur une partie quelconque du corps. La composition renfermant cet extrait peut être appliquée le matin et/ou le soir, sur l'ensemble du visage, du cou, des mains et éventuellement du décolleté voire du corps.

La composition mise en œuvre selon l'invention comprend généralement, outre l'extrait décrit précédemment, un milieu physiologiquement acceptable et de préférence cosmétiquement acceptable, c'est-à-dire qui convient à une utilisation en contact avec la peau humaine sans risque de toxicité, d'incompatibilité, d'instabilité, de réponse allergique et notamment qui ne provoque pas de sensations d'inconfort (rougeurs, tiraillements, picotements.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau-dans-huile ou huile-dans-eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée ou des eaux florales), un alcool tel que l'éthanol.

Ladite composition cosmétique peut également comprendre, en outre de l'extrait selon l'invention :
- au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.
- un ou plusieurs agent(s) humectants, tels que les polyols (glycérine, diglycérine, le propanediol, le caprylyl glycol, le pentylène glycol, l'hexanediol), les sucres, les glycosaminoglycanes tels que l'acide hyaluronique et ses sels et esters; et les polyquaterniums tel que le lipidure PMB. Ledit agent humectant sera présent dans la composition à une teneur de l'ordre de 0 à 30%, de préférence 0.005 à 10% en poids total de la composition.
- Un ou plusieurs agent(s) émollient(s) qui peuvent être choisi (s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglyceride), les beurres tels que les beurres de karité (butyrospernum parkii butter extract, shea butter ethyl esters, commercialisés sous les noms de LIPEX SHEASOFT, LIPEX SHEA-U, LIPEX SHEA, LIPEX SHEALIGHT, LIPEX SHEA TRIS) ou de moringa (moringa oil/hydrogenated moringa oil esters), les cires (acacia decurrens flower wax & helianthus annuus cera seed seed wax, C10-18 triglycerides), les huiles végétales, le phytosqualane, les alcanes (undecane, tridecane). Ledit agent émollient sera présent dans la composition à une teneur de l'ordre de 0,1 à 30%, de préférence 0,5 à 10% en poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (commercialisés sous les noms ARISTOFLEX AVC, Aristoflex AVS, Aristoflex HMB, SIMULGEL NS, Simulgel EG, Simulgel 600, Simulgel 800, Pemulen, carbopol, Sepiplus 400, Seppimax zen, Sepiplus S, COSMEDIA SP). Ledit agent gélifiant et/ou épaississant sera présent dans la composition à une teneur de l'ordre de 0,1 à 10% en poids total de la composition.
- Un ou plusieurs agent(s) tensioactif(s), tels que les lecithines, les dérivés de polyglycérol, les dérivés de sucre (les dérivés de glucosides ou de xylosides commercialisés sous le nom MONTANOV 68, MONTANOV 202, Montanov 82, MONTANOV L, EASYNOV), les phosphates (C20-22 alkyl phosphate commercialisé sous le nom SENSANOV WR). Ledit agent tensioactif, sera présent dans une teneur de l'ordre de 0,1 à 8%, de préférence 0,5 à 3% en poids, par rapport au poids total de la composition.
- Un ou plusieurs agent(s) actif(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle), la vitamine A et ses dérivés, la vitamine E et ses dérivés, la vitamine B3 ou Niacinamide, le panthénol, les oligo éléments, l'allantoïne, l'adenosine, les peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5 commercialisés sous le nom NP RIGIN, MATRIXYL 3000, IDEALIFT, EYESERYL), les extraits végétaux (glycyrrhiza glabra extract, centella asiatica leaf extract, secale cereale seed extract), les extraits de levures, les alpha hydroxyacides tels que l'acide glycolique ou lactique, l'acide tranexamique et ses dérivés tel que le cetyl tranexamique ester etc. Ledit agent actif sera présent dans la composition à une teneur de l'ordre de 0,1 à 10% en poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention a également pour objet l'utilisation cosmétique de l'extrait de fleur de *Camellia japonica* tel que décrit précédemment ou de la composition cosmétique précédemment décrite pour apaiser et/ou réduire l'inflammation la peau humaine.

Dans ce mode de réalisation, l'extrait ou la composition est appliqué sur une peau irritée non pathologique.

L'invention sera maintenant illustrée par les exemples non limitatifs suivants.

### Exemple 1 : préparation d'un extrait hydro-alcoolique de Camellia japonica alba plena conforme à l'invention

### Etape i.

On a tout d'abord procédé à l'extraction huileuse de fleurs de Camellia dans les conditions décrites dans l'exemple 1 de la demande WO2010112760.

### Etape ii.

La poudre résiduelle de fleurs obtenue à l'issue de l'étape i. est mélangée en proportion massique 1 :8 dans un mélange constitué de 80% de propanediol et 20 % d'eau qualité analytique. Le mélange est réalisé dans un réacteur en inox muni d'un agitateur à hélices. La poudre est dispersée dans le mélange d'extraction à température ambiante pendant 45 min dans le réacteur fermé sous agitation et sous flux d'azote.

### Etape iii.

Le mélange est ensuite soumis à une cavitation ultrasonore à 25 kHz pendant 30 min dans un réacteur fermé sous flux d'azote.

### Etape iv.

Le mélange est ensuite laissé dans le réacteur fermé sous flux d'azote sans agitation pendant 2 heures à température ambiante.

### Etape v.

Après l'étape de macération iv., le mélange est essoré sur une toile filtrante de porosité 1 micron. L'extrait hydro-alcoolique est ensuite filtré sur un filtre à plaques muni de plaques de porosité 0,2 microns. L'essorage et la microfiltration sur plaque sont conduites à température ambiante.

L'extrait hydro-alcoolique final est limpide, fluide, homogène, de couleur jaune orangée et d'odeur fleurie. Il doit être conservé au froid jusqu'à son utilisation.

### Exemple 2 : Inhibition de voies liées à l'inflammation dans des kératinocytes humains normaux traités avec l'extrait de Camellia obtenu à l'exemple 1

**Protocole** : Des kératinocytes épidermiques humains normaux issus de trois donneurs différents ont été ensemencés en plaque 24 puits et cultivés en milieu complémenté kératinocyte-SFM (k-SFM) pendant 48h à 37°C et 5% de CO₂. Les cellules ont ensuite été incubées avec ou sans (condition non traitée) 0,1% d'extrait de Camellia préparé à l'exemple 1 pendant 48h. Chaque condition a été effectuée en duplicat. Les ARN totaux ont été extraits à l'aide de TriPure Isolation Reagent^{®} selon le protocole préconisé par le fournisseur. Les ADN complémentaires ont été synthétisés et un transcriptome a été réalisé sur puce Affymetrix GeneChip Human Transcriptome Array 2.0. L'analyse bioinformatique des gènes dont l'expression est modulée *a minima* par un facteur 2 a été effectuée avec le logiciel Ingenuity Pathway Analysis (IPA^{®}, QIAGEN). Ce logiciel recueille des informations sur les interactions molécule à molécule, les réseaux biologiques et les voies canoniques dans la base de données Ingenuity Knowledge.

**Résultats :** Les fonctions cellulaires significativement inhibées par l'extrait hydro-alcoolique de Camellia selon l'invention sont majoritairement des voies impliquées dans la réponse immunitaire inflammatoire. Les gènes impliqués dans ces différentes voies ainsi que leur niveau d'inhibition par l'extrait hydro-alcoolique de Camellia selon l'invention *versus* témoin non traité (niveau d'expression > 2) sont reportés dans le tableau I.

**Tableau I : Liste des gènes impliqués dans les voies liées à l'inflammation dont l'expression est diminuée par l'extrait hydro-alcoolique de Camellia selon l'invention.**

| **Symbole** | **Nom du gène** | **Niveau d'expression (*vs* témoin non traité)** |
|---|---|---|
| B2M | beta-2-microglobulin | -10,500 |
| HLA-B | major histocompatibility complex, class I, B | -10,150 |
| IL1A | interleukin 1 alpha | -2,580 |
| SERPINE1 | serpin family E member 1 | -2,800 |
| ACVR1B | activin A receptor type 1B | -2,690 |
| CEBPB | CCAAT enhancer binding protein beta | -2,380. |
| TNF | tumor necrosis factor | -2,120 |
| HLA-DQB1 | major histocompatibility complex, class II, DQ beta 1 | -2,190 |

*Les analyses ont été réalisées sur IPA avec un cutoff de 2 : toutes les cibles avec un niveau d'expression <-2 ou >2 sont prises en compte dans l'analyse et considérées comme modulées significativement effectivement.*

Le HLA-B fait partie du complexe majeur d'histocompatibilité et B2M est connu comme étant associé à ce complexe qu'il participe également à réguler.

Les kératinocytes contribuent à l'initiation de la réponse inflammatoire *via* la sécrétion de cytokines pro-inflammatoires telles que le TNFα et l'IL1α. Ils jouent aussi un rôle dans l'immunité cutanée. En effet, ils expriment des molécules du majeur d'histocompatibilité (B2M, HLA-B, HLA-DQB1, ...) impliqué dans la présentation des antigènes aux cellules immunitaires effectrices. D'autre part, avec l'âge et les différents stress auxquels est soumise la peau, certaines cellules deviennent capables de sécréter des messagers de stress (ex : interleukine pro-inflammatoire IL1α, plasminogen activator inhibitor-1 (PAI1) codé par le gène SERPINE1, CEPBβ...) générant un environnement cutané inflammatoire.

Par sa capacité à inhiber l'expression de différents gènes impliqués dans **l'inflammation** cutanée, l'extrait hydro-alcoolique de Camelia blanc selon l'invention présente un potentiel cosmétique **apaisant.**

### EXEMPLE 3 : Composition cosmétique

Les compositions suivantes peuvent être préparées de façon classique pour l'homme du métier. Les quantités indiquées ci-dessous sont exprimées en pourcentages pondéraux. Les ingrédients sont identifiés conformément à la dénomination INCI.

### A - émulsion huile/eau gel

| **Nom INCI** | **(% W/W)** |
|---|---|
| Limnanthes alba (meadowfoam) seed oil | 1-10 |
| Butyrospermum parkii butter (LIPEX SHEASOFT) | 1-10 |
| Butyrospernum parkii butter extract (LIPEX SHEA TRIS) | 1-10 |
| Camellia oleifera seed oil | 1-10 |
| Cetyl ethylhexanoate | 1-5 |
| Squalane | |
| Ammonium acryloyldimethyltaurate/vp copolymer | 0.1-5 |
| ACRYLATES/C10-30 ALKYL ACRYLATE CROSSPOLYMER | 0.1-2 |
| Xanthane gum | 0.01-5 |
| ORYZA SATIVA (RICE) POWDER | 0.1-5 |
| Sodium hyaluronate | 0.01-3 |
| Glycerin | 1-30 |
| Polyquaternium-51 | 1-10 |
| Adenosine | 0.1-0.5 |
| Niacinamide | 0.1-5 |
| Palmitoyl Tetrapeptide-7 | 1-5 |
| Secale cereale (rye) seed extract | 0.1-5 |
| Tocopheryl acetate | 0.1-5 |
| Extrait hydro-alcoolique de fleurs de *Camellia japonica* selon l'invention | 0.001-10 |
| Yeast extract | 0.1-5 |
| Glycyrrhiza Glabra extract | 0.1-5 |
| Glycols (Caprylyl Glycol and/or Pentylene Glycol and/or Butylene Glycol and/or propanediol) | 0,1-10 |
| Water | Qs 100 |

### b - émulsion huile/eau crème

| **Nom INCI** | **(% w/w)** |
|---|---|
| Jojoba esters | 1-5 |
| Limnanthes alba (meadowfoam) seed oil | 0.1-5 |
| C8-12 ACID TRIGLYCERIDE | 1-5 |
| Lauroyl lysine | 1-5 |
| Camellia oleifera seed oil | 1-10 |
| Phytosteryl/octyldodecyl lauroyl glutamate | 1-5 |
| Squalane | 1-10 |
| Ammonium acryloyldimethyltaurate/vp copolymer | 1-5 |
| Polyglyceryl-6 distearate & jojoba esters & polyglyceryl-3 beeswax & cetyl alcohol | 1-7 |
| Xanthan Gum | 0,01-2 |
| Centella asiatica leaf extract | 0.1-5 |
| Adenosine | 0.1-0.5 |
| Niacinamide | 0.1-5 |
| Secale cereale (rye) seed extract | 0.1-5 |
| Palmitoyl tripeptide-1 & palmitoyl tetrapeptide-7 | 1-5 |
| Tranexamic cetyl ester | 0.001-5 |
| Ascorbyl glucoside | 0.001-5 |
| Yeast extract | 1-3 |
| Saccharide isomerate | 1-5 |
| Extrait hydro-alcoolique de fleurs de *Camellia japonica* selon l'inventions | 0.001-10 |
| Glycyrrhiza Glabra extract | 0.001-5 |
| Water | Qs 100 |

Ces compositions peuvent être appliquées tous les jours, matin et/ou soir, sur la peau.

## Revendications

1. Procédé de préparation d'un extrait hydro-alcoolique de fleurs de *Camellia japonica,* comprenant les étapes suivantes :
i. l'imprégnation d'une poudre de fleurs de Camellia par un corps gras ou un mélange de corps gras à une température supérieure à la température de fusion dudit corps gras et sous atmosphère dépourvue ou essentiellement dépourvue d'oxygène, de préférence une huile, puis séparation pour obtenir d'une part un extrait huileux de fleurs de Camellia et d'autre part une poudre résiduelle des fleurs ainsi extraites,
ii. l'imprégnation de la poudre résiduelle des fleurs obtenue à l'étape i. au moyen d'un solvant d'extraction à base d'un mélange d'alcool et d'eau sous atmosphère dépourvue ou essentiellement dépourvue d'oxygène,
iii. l'extraction de la dite poudre résiduelle de fleurs par ultrasons et sous atmosphère dépourvue ou essentiellement dépourvue d'oxygène,
iv. la macération de la dite poudre dans le solvant d'extraction et sous atmosphère dépourvue ou essentiellement dépourvue d'oxygène,
v. la clarification de l'extrait hydro-alcoolique de fleurs de Camellia.

2. Procédé selon la revendication précédente, caractérisé en ce les étapes i., ii., iii., et iv. sont opérées sous atmosphère d'azote.

3. Procédé selon l'une quelconque des revendications précédentes, **caractérisé en ce que** la poudre de fleurs de *Camellia japonica* est sous la forme d'un produit dispersible obtenu par broyage à une température comprise entre -20 et - 80°C.

4. Procédé selon l'une quelconque des revendications précédentes, **caractérisé en ce que** l'étape i est opérée avec un corps gras ou un mélange de corps gras à une température supérieure à la température de fusion dudit corps gras ou dudit mélange et sous atmosphère dépourvue ou essentiellement dépourvue d'oxygène.

5. Procédé selon l'une quelconque des revendications précédentes, **caractérisé en ce que** la fleur de Camellia provient de la variété *Camellia japonica alba plena.*

6. Procédé selon l'une quelconque des revendications précédentes, **caractérisé en ce que** le ou les corps gras mis en œuvre à l'étape i. est une huile végétale liquide à température ambiante, un beurre végétal présentant un point de fusion compris entre 25 et 40°C, ou une cire végétale présentant un point de fusion supérieur à 40°C, et de préférence, le ou les corps gras est une huile choisie parmi l'huile de camélia, l'huile de colza, l'huile de tournesol, l'huile d'olive, l'huile de sésame, l'huile de noyaux d'abricot, l'huile de pépin de raisin, l'huile d'amande douce, l'huile de carthame, l'huile de noisette, l'huile d'argan, l'huile de rosier muscat, l'huile d'onagre, l'huile de bourrache, la cire liquide de jojoba, et leurs mélanges.

7. Procédé selon l'une quelconque des revendications précédentes, **caractérisé en ce que** le solvant d'extraction mis en œuvre à l'étape ii. est un mélange de polyol et d'eau, et de préférence de propanadiol et d'eau.

8. Procédé selon la revendication précédente, **caractérisé en ce que** le ratio volumique alcool:eau du solvant d'extraction mis en œuvre à l'étape ii. est compris entre 95/5 et 50/50, de préférence entre 90/10 et 70/30, et plus préférentiellement entre 85/15 et 75/25.

9. Procédé selon la revendication précédente, **caractérisé en ce que** l'étape iii. d'extraction assistée par ultrasons est opérée pendant une durée comprise entre 15 et 60 minutes, de préférence entre 30 et 40 minutes, à une fréquence de cavitation inférieure à 100 kHz, de préférence comprise entre 20 et 30 kHz.

10. Procédé selon la revendication précédente, **caractérisé en ce que** l'étape v. de clarification est opérée par filtration, de préférence en deux temps :
- une première microfiltration centrifuge sur une toile filtrante de porosité inférieure à 5 microns et de préférence inférieure ou égale à 1 micron, puis
- une seconde microfiltration clarifiante sur une plaque de filtration de porosité inférieure à 1 micron et de préférence inférieure ou égale à 0,2 micron.

11. Procédé selon l'une quelconque des revendications précédentes, **caractérisé en ce que** les étapes i., ii., iii., et/ou iv. sont conduites en l'absence de lumière ou de tout rayonnement oxydant tel que les UV.

12. Extrait hydro-alcoolique de fleurs de *Camellia japonica,* comprenant majoritairement un mélange de sucres, de camellioside A et de caméllioside B.

13. Extrait hydro-alcoolique selon la revendication précédente, **caractérisé en ce que** les sucres, et les camelliosides sont présents en une proportion massique d'environ 2 :1 dans l'extrait.

14. Extrait hydro-alcoolique selon l'une quelconque des revendications 12 ou 13, **caractérisé en ce qu'**il est obtenu au moyen d'un procédé selon l'une quelconque des revendications 1 à 11.

15. Extrait hydro-alcoolique selon l'une quelconque des revendications 13 à 15, **caractérisé en ce que** la fleur de Camellia provient de la variété *Camellia japonica alba plena.*

16. Composition cosmétique comprenant, dans un milieu physiologiquement acceptable, au moins un extrait de fleur de Camellia japonica selon l'une quelconque des revendications 12 à 14.

17. Utilisation cosmétique non thérapeutique d'un extrait de fleur de Camellia japonica selon l'une quelconque des revendications 12 à 14, comme actif apaisant et/ou anti-inflammatoire.
